Europäisches Patentamt

European Patent Office

Office européen des brevets

(19)

(11) Veröffentlichungsnummer: **0 212 069 B1**

(12) **EUROPÄISCHE PATENTSCHRIFT**

(45) Veröffentlichungstag der Patentschrift: 03.10.90

(21) Anmeldenummer: **86106272.7**

(22) Anmeldetag: **07.05.86**

(51) Int. Cl.⁵: **A 61 K 6/04, C 22 C 5/04**

(54) Verwandlung von silber- und goldfreien Palladiumlegierungen zum Aufbrennen von Dentalkeramiken.

(30) Priorität: **24.06.85 DE 3522523**

(43) Veröffentlichungstag der Anmeldung:
**04.03.87 Patentblatt 87/10**

(45) Bekanntmachung des Hinweises auf die
Patenterteilung:
**03.10.90 Patentblatt 90/40**

(84) Benannte Vertragsstaaten:
**AT BE CH DE FR GB IT LI LU NL SE**

(56) Entgegenhaltungen:
**EP-A-0 124 750**
**DE-A-3 146 794**
**DE-C-3 239 338**
**DE-C-3 406 711**
**US-A-4 387 072**

(73) Patentinhaber: **Degussa Aktiengesellschaft**
**Weissfrauenstrasse 9**
**D-6000 Frankfurt am Main 1 (DE)**

(72) Erfinder: **Groll, Werner, Dr.**
**Kardinal Döpfner Str. 5**
**D-8757 Karstein (DE)**
Erfinder: **Schöck, Gernot**
**Bahnhofstrasse 56**
**D-6451 Bruchköbel (DE)**
Erfinder: **Hathaway, Doris**
**Lahnstrasse 20**
**D-6450 Hanau 7 (DE)**
Erfinder: **Wagner, Rudolf, Dr.**
**Breslauer Str. 10**
**D-7537 Renschingen 3 (DE)**

Courier Press, Leamington Spa, England.

**Beschreibung**

Die Erfindung betrifft die Verwendung von gold- und silberfreien Palladiumlegierungen als Werkstoffe zum Aufbrennen von Dentalkeramiken.

Die komplexen Anforderungen, die an festsitzenden Zahnersatz gestellt werden, sind die Ursache dafür, daß Kronen und Brücken in großem Umfang aus Metall gefertigt werden. Metallisacher Zahnersatz wird vorwiegend aus ästhetischen Gründen mit Dentalkeramik verblendet, so daß die Legierungen mit den gängigen Dentalkeramiksorten kompatibel sein müssen. Für diese Anwendungsfälle haben sich hochgoldhaltige Legierungen mit ca. 60—90% Gold, ca. 1—15% Palladium, ca. 1—15% Platin, Zusätze von Silber, Zinn, Indium und/oder Eisen und kornfeinende Elementen wie Iridium, Ruthenium und/oder Rhenium sehr gut bewährt. Die hohen Edelmetallpreise und die Kostenexplosion im Gesundheitswesen haben jedoch in den letzten Jahren — nicht zuletzt auf Grund gesetzgeberischer Maßnahmen — dazu geführt, daß verstärkt goldreduzierte Legierungen entwickelt werden mussten. Solche Legierungen auf Gold-Palladium- und Gold-Palladium-Silber-Basis enthalten jedoch immer noch ca. 50—60% Gold. Silberhaltige Legierungen verursachen dabei eine Grünverfärbung der Dentalkeramik beim Aufbrennprozeß und werden nur selten in der Praxis verwendent. Auch goldarme Palladiumlegierungen, die 20 bis 35% Gold, 45 bis 65% Palladium, 6 bis 15% Kupfer, 0 bis 10% Nickel, 0 bis 12% Indium, 0 bis 12% Zinn, 0 bis 4% Gallium und 0 bis 1% Tantal enthalten (DE—C—3 406 711), sind noch zu teuer und sich auch in ihren Werkstoffeigenschaften nicht optimal.

In den letzten Jahren sind daher silberfreie Palladium-Basislegierungen entwickelt worden, die wegen der geringen Dichte und des günstigen Preises des Palladium den preisgünstigen Typ einer Edelmetallaufbrennlegierung darstellen. Eine weitere Preisreduzierung ist nur noch durch eine Vermindung des Palladiumgehaltes möglich. Legierungen auf der Basis Palladium-Kobalt (z.B. DE—A—33 24 987 und US—A—4 261 744) zeigen insbesondere in Bereichen geringer Palladiumkonzentrationen (ca. 75% und weniger) Blasenbildung und Verfärbung in der Verblendkeramik. Die Entstehung der Blasen kann zwar durch Zugabe von Zink. Aluminium oder Silizium unterdrückt werden, doch zeigen diese Legierungen dann Umwandlungen in festem Zustand, wodurch die Paßgenauigkeit des Zahnersatzes beeinträchtigt werden kann.

Es sind weiterhin eine Vielzahl von Palladium-Kupfer-Legierungen mit einem oder mehreren der Legierungselemente Zinn, Indium, Kobalt und kornfeinenden Zusätzen von Ruthenium und Rhenium bekannt (z.B. DE—A—33 16 595, DE—A—32 44 802, DE—A—31 46 794, DE—A—32 39 338, DE—A—32 47 398). Alle diese Legierungen enthalten zusätzlich Gallium als Legierungsbestandteil, da dieses die Gießbarkeit der Legierungen deutlich verbessert. Verzichtet man bei diesen Legierungen auf das Gallium, so sind die Gußergebnisse nur schwer reprodzierbar und es kommt haüfig zur Lunkerbildung. Gallium erniedrigt die Solidustemperatur stark und weitet das Schmelzintervall auf, wodurch die Warmfestigkeit beim Keramikbrand verringert und die Gefahr von Seigerungen bei der Erstarrung erhöht werden. Gallium bewirkt außerdem eine starke Härtesteigerung. Galliumhaltige Legierungen weisen ein heterogenes Gefüge auf und sind daher korrosionsanfälliger. Eine erhöhte Neigung zur Blasenbildung in der ausgebrannten Keramik wird, speziell bei geringen Palladium-Gehalten, ebenfalls beobachtet.

Die bekannten Palladium-Kupfer-Basis-Legierungen besitzen meist einen thermischen Ausdehnungskoeffizienten kleiner $14{,}0 \times 10^{-6}$/K, so daß die Kompatibilität mit neueren, höher expandierenden Dentalkeramiksorten nicht mehr gewährleistet ist.

Es war daher Aufgabe der vorliegenden Erfindung, gold- und silberfreie Paladiumlegierungen als Werkstoff zum Aufbrennen von Dentalkeramiken zu entwickeln, die möglichst billig, extrahart und kompatibel mit den bekannten Dentalkeramiken sein sollten. Außerdem sollten sich auch ohne Galliumzusätze gut verarbeitbar sein und eine Solidustemperatur von mehr als 1150°C aufweisen.

Diese Aufgabe wurde erfindungsgemäß gelöst durch die Verwendung von Legierungen, bestehend aus 60 bis 80% Palladium, 0 bis 5% Platin, 0 bis 1% Ruthenium und/oder Rhenium, 2 bis 20% Kupfer, je 1 bis 12% Zinn und/oder Indium, 0,2 bis 5% Wolfram und 0 bis 15% Kobalt, wobei die Summe der Gehalte an Zinn und Indium zwischen 5 und 14% liegen muß.

Vorzugsweise verwendet man Palladiumlegierungen, die 0,5 bis 2,5% Wolfram enthalten. Besonders bewährt haben sich silberfreie Palladiumlegierungen mit 65 bis 75% Palladium, 10 bis 15% Kupfer, 5 bis 10% Zinn, 2 bis 6% Indium, 0,1 bis 1% Ruthenium und/oder Rhenium und 0,5 bis 2,5% Wolfram, wobei die Summe der Gehalte an Zinn und Indium zwischen 10 und 13% liegen sollte.

Um möglichst billige Palladium-Legierungen zu entwickeln, mußten geeignete Nichtedelmetalle gefunden werden, die die technischen Eigenschaften und die Verarbeitbarkeit des Palladiums auch bei Zugabe höherer Konzentrationen nicht zu stark verändern. Da Nickel wegen seiner bekannten gesundheitsbeeintrachtigenden Risiken nicht in einer Dentallegierung verwendet werden soll, Eisen, Chrom und Mangan die Eigenschaften deutlich verschlechtern, stehen lediglich Kupfer und Kobalt als Hauptlegierungselemente zur Verfügung, die mit Palladium eine durchgehende Mischkristallreihe bilden. Die Härte steigt bei Zugabe bis zu 20%, Kupfer oder Kobalt nicht über 130 HV 5 an, das Schmelzintervall wird vergleichsweise nur gering abgesenkt. Kobalt führt jedoch zu einer starken Anhebung des thermischen Ausdehnungskoeffizienten und zu einer intensiven dunklen, bläulichgrauen Oxidfarbe, weshalb der Kobalt-Gehalt 15 Gew.% nicht überschreiten sollte. Bei Kupfergehalten von mehr als 20% verzundert die Legierung, das Oxid hat keine feste Bindung mehr zum Metall. Durch Zugabe von 1—12%

2

Zinn und/oder 1—12% Indium in unterschiedlichen Konzentrationsverhältnissen konnte der Palladium-Gehalt weiter reduziert und die technischen Eigenschaften entsprechend den eine extraharte Aufbrennlegierung gestellten Anforderung erreicht werden. Es zeigte sich jedoch, daß speziell bei Palladiumgehalten kleiner 75% eine zu hohe Konzentration an Zinn und Indium zur Bildung von Zinn- und indiumreichen Phasen und zu einem starken Härteanstieg führt. Legierungen mit hohem Zinn- und Indium-Gehalt waren zudem häfig gußbrüchig. Diese nachteiligen Auswirkungen von Zinn und Indium können aber dan vermeiden werden, wenn die Summe der Gehalte an Zinn und Indium 14% nicht übersteigt.

Alle diese Legierungen im Zusammensetzungsbereich 60—80% Pd, 2—20% Cu, 0—15% Co, 1—12% Sn, 1—12% In zeigten jedoch Mängel bezüglich der Verarbeitbarkeit.

Die hochkupferhaltigen Legierungen lieferten nur schwer reproduzierbare Gußergebnisse, da diese Legierungen empfindlich auf kleine Veränderungen der Gießbedingungen reagieren. Die Angußtrichter waren stets blasig und nach außen gewöhlt, die Gußobjekte zeigten teilweise Lunker. Bei der Verblendung mit Dentalkeramik traten Blasen in der Grundmasse und Verfärbung auf. Die kobalthaltigen Legierungen zeigten zwar ein besseres Gießverhalten, doch war die Blasenbildung bei der Keramikverblendung noch ausgeprägter als bei den kupferreichen Legierungen. Vermutlich nehmen die Legierungen während des Schmelzens Gase auf, die beim Erstarren bzw. beim Keramikbrand wieder ausgeschieden werden.

Überraschenderweise hat sich nun gezeigt, daß Wolfram in Konzentrationen von 0,2—5 Gew.% die oben beschriebenen Mängel bezüglich der Verarbeitbarkeit beseitigt, ohne daß die technischen Eigenschaften negativ beeinflußt werden. Die Güsse der kupferreichen Legierungen sind lunkerfrei, die Ergebnisse reproduzierbar. Bei der Verblendung mit Dentalkeramik wird weder keine Blasenbildung mehr beobachtet, die grüne Verfärbung der Einbettmasse und der Dentalkeramik wird unterdrückt. Auch die kobaltreichen Legierungen lassen sich bei einem Wolframgehalt von 0,2—5% blasenfrei verblenden. Die Haftung zwischen den wolframhaltigen Legierungen und der Dentalkeramik ist sehr gut. Diese Legierungen sind wegen ihres hohen thermischen Ausdehungskoeffizienten (größer $14,0 \times 10^{-6}$/K) mit allen Dentalkeramiksorten, auch mit höherexpandierenden, kompatibel.

Der Einsatz dieser Legierungen ist nicht auf keramikverblendeten, festsitzenden Zahnersatz beschränkt. Die Legierungen eignen sich ebenfalls hervorragend für unverblendeten bzw. für mit Kunststoff verblendeten Zahnersatz. Eine Verwendung für die Ätzbrückentechnik ist ebenfalls möglich.

Die folgende Tabelle zeigt die Zusammensetzung einiger erfindungsgemäßer Legierungen und ihre wesentlichen Eigenschaften. Dabei werden diese Legierungen (7 bis 9) mit bekannten Legierungen (1 bis 6) verglichen).

| Legierung Nr. | Zusammensetzung in Massen - % | | | | | | | | | | Schmelz-intervall [°C] | Guß-härte [HV5] | 0,2% Dehn-grenze [Nmm⁻²] | Ausdehnungs-koeffizient RT-600°C x10⁻⁶k⁻¹ | Gieß-verhalten | Ver-blend-verhalten |
|---|---|---|---|---|---|---|---|---|---|---|---|---|---|---|---|---|
| | Au | Pd | Ru | Co | Cu | Ga | In | Si | Sn | W | | | | | | |
| 1 | - | 69,5 | 0,5 | 19,5 | - | - | 5 | 0,2 | 5,3 | - | 1220-1180 (635-680°C) (therm. Effekt) | 320 | - | 15,3 | gut | Blasen |
| 2 | 2 | 78,5 | 0,4 | - | 10 | 9,1 | - | - | - | - | 1185-1065 | 405 | - | 13,6 | gut | " |
| 3 | - | 73,2 | 0,3 | - | 15 | 1,5 | 5 | - | 5 | - | 1270-1145 | 260 | - | 13,7 | gut | " |
| 4 | - | 69,5 | 0,5 | - | 13,5 | - | 9 | - | 7,5 | - | 1220-1170 | 315 | - | 14,6 | Brüche d.Gußob-jekte | " |
| 5 | - | 69,5 | 0,5 | - | 19,5 | - | 5 | - | 5,5 | - | 1225-1175 | 235 | 485 | 14,2 | Gußkegel m.Blasen z.T.Lunker | " |
| 6 | - | 69,5 | 0,5 | 12 | 8 | - | 10 | - | - | - | 1230-1170 | 290 | - | 15,5 | gut | " |
| 7 | - | 72 | 0,5 | 2 | 12,5 | - | 5 | - | 7 | 1 | 1285-1200 | 230 | 515 | 14,2 | gut | gut |
| 8 | - | 72 | 0,5 | 2 | 13,5 | - | 3 | - | 8 | 1 | 1285-1190 | 230 | 500 | 14,4 | gut | gut |
| 9 | - | 77,5 | 0,5 | 7 | 3 | - | 10 | - | 1 | 1 | 1320-1240 | 260 | - | 14,4 | gut | gut |

EP 0 212 069 B1

**Patentansprüche**

1. Verwendung von gold- und silberfreien Palladiumlegierungen, bestehend aus 60 bis 80% Palladium, 0 bis 5% Platin, 0 bis 1% Ruthenium und/oder Rhenium, 2 bis 20% Kupfer, je 1 bis 12% Zinn und/oder Indium, 0,2 bis 5% Wolfram und 0 bis 15% Kobalt, wobei die Summe der Gehalte an Zinn und Indium zwischen 5 und 14% liegen muß, als Werkstoffe zum Aufbrennen von Dentalkeramiken.

2. Verwendung von gold- und silberfreien Paladiumlegierungen nach Anspruch 1, die 0,5 bis 2,5% Wolfram enthalten.

3. Verwendung von gold- und silberfreien Palladiumlegierungen nach Anspruch 1, bestehend aus 65 bis 75% Palladium, 10 bis 15% Kupfer, 5 bis 10% Zinn, 2 bis 6% Indium, 0,1 bis 1% Ruthenium und/oder Rhenium und 0,5 bis 2,5% Wolfram, wobei die Summe der Gehalte an Zinn und Indium zwischen 10 und 13% liegt.

**Revendications**

1. Utilisation d'alliages de palladium exempts d'or et d'argent, constitués de 60 à 80% de palladium, de 0 à 5% de platine, de 0 à 1% de ruthénium et/ou de rhénium, de 2 à 20% de cuivre, de 1 à 12% chacun d'étain et/ou d'indium, de 0,2 à 5% de tungstène et de 0 à 15% de cobalt, la somme des teneurs en étain et en indium devant être comprise entre 5 et 14%, en tant que matériaux pour l'application par cuisson de céramiques dentaires.

2. Utilisation d'alliages de palladium exempts d'or et d'argent selon la revendication 1, qui contiennent de 0,5 à 2,5% de tungstène.

3. Utilisation d'alliages de palladium exempts d'or et d'argent selon la revendication 1, constitués de 65 à 75% de palladium, de 10 à 15% de cuivre, de 5 à 10% d'étain, de 2 à 6% d'indium, de 0,1 à 1% de ruthénium, et ou de rhénium et de 0,5 à 2,5% de tungstène, la somme des teneurs en étain et indium étant comprise entre 10 et 13%.

**Claims**

1. The use of gold- and silver-free palladium alloys consisting of 60 to 80% palladium, 0 to 5% platinum, 0 to 1% ruthenium and/or rhenium, 2 to 20% copper, 1 to 12% tin and/or 1 to 12% indium, 0.2 to 5% tungsten and 0 to 15% cobalt, the sum of the tin and indium contents having to be between 5 and 14%, as materials for the firing on of dental ceramics.

2. The use of gold- and silver-free palladium alloys as claimed in claim 1, the alloys containing 0.5 to 2.5% tungsten.

3. The use of gold- and silver-free palladium alloys as claimed in claim 1, the alloys consisting of 65 to 75% palladium, 10 to 15% copper, 5 to 10% tin, 2 to 6% indium, 0.1 to 1% ruthenium and/or rhenium and 0.5 to 2.5% tungsten, the sum of the tin and indium contents being between 10 and 13%.